(19) Europäisches Patentamt
European Patent Office
Office européen des brevets

(11) **EP 2 210 604 A1**

(12) **EUROPEAN PATENT APPLICATION**
published in accordance with Art. 153(4) EPC

(43) Date of publication:
**28.07.2010 Bulletin 2010/30**

(21) Application number: **08834122.7**

(22) Date of filing: **08.09.2008**

(51) Int Cl.:
*A61K 31/415* (2006.01)    *A61K 31/5415* (2006.01)
*A61K 31/519* (2006.01)    *A61P 19/06* (2006.01)
*A61P 29/00* (2006.01)

(86) International application number:
**PCT/MX2008/000123**

(87) International publication number:
**WO 2009/041798 (02.04.2009 Gazette 2009/14)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **26.09.2007 MX 2007011927**

(71) Applicant: **Espinosa Abdala, Leopoldo de Jesús**
**C.P. 45110 Zapopan, Jalisco (MX)**

(72) Inventors:
• **GARCÍA ARMENTA, María Elena**
**C.P. 45020 Guadalajara**
**Jalisco (MX)**

• **SANTOS MURILLO, Josefina**
**C.P. 44600 Zapopan**
**Jalisco (MX)**
• **ÁLVAREZ OCHOA, Víctor Guillermo**
**C.P. 45222 Zapopan**
**Jalisco (MX)**

(74) Representative: **Carvajal y Urquijo, Isabel et al**
**Clarke, Modet & C.**
**C/ Goya, 11**
**E-28001 Madrid (ES)**

(54) **PHARMACEUTICAL COMPOSITIONS COMBINING A NONSTEROIDAL ANTI-INFLAMMATORY AGENT AND A XANTHINE OXIDASE INHIBITING AGENT, WHICH CAN BE USED TO CONTROL AND TREAT GOUT, GOUTY ARTHRITIS AND RELATED DISEASES**

(57)    The invention relates to a pharmaceutical composition comprising the synergic combination of a nonsteroidal anti-inflammatory agent known as meloxicam or celecoxib and a xanthine oxidase inhibiting agent know as allopurinol, as well as pharmaceutically acceptable excipients, which are formulated in a single dosage unit to be administered orally. The compositions, which are used to control and treat gouty disease or gout, gouty arthritis and other related diseases, provide an improved therapeutic effect and faster pharmacological action in less time, reducing pain, inflammation and plasma urate levels, as well as providing reductions in the doses administered, the risk of severe complications and the risk of side effects.

**EP 2 210 604 A1**

**Description**

## FIELD OF INVENTION

The present invention has application in pharmaceutical industry and discloses several pharmaceutical compositions comprising a synergistic combination of a non-steroidal anti-inflammatory agent, such as active principle: Meloxicam or Celecoxib and a xanthine oxidase enzyme inhibiting agent, such as active principle: Allopurinol, in addition to pharmaceutically acceptable excipients; which are formulated in a single dosage unit to be orally administered, those which are indicated for control and treatment of gout, gouty arthritis and related diseases.

The combination of above mentioned active principles produces a higher synergistic effect when administered in a single dosage unit unlike when these are independently administered, causing benefits such as: administration of lower active principle concentrations comprised in the formulation, smaller administered doses, faster pharmacological action, therapeutic effect enhancement and lower risks of showing,side effects.

## BACKGROUND OF INVENTION

Gout or gouty disease is a metabolic disease produced by an accumulation of uric acid in the body, mainly in joints and in kidney, thus being considered a rheumatic disease.

Gout is a predominant disease in men (>95%), and is seldom noticed in women but is present along menopause.

Gouty disease comprises a group of heterogeneous disorders which are isolated or jointly present such as: 1) Hyperuricemia; 2) Acute monoarticular inflammatory arthritis attacks, which are usually present in metatarsophalangealal joints, knees, ankle and other joints causing inflammation and intense pain; 3) Deposits in form of gout tophii presented with a urate crystal pool surrounded by an intense inflammatory reaction formed by macrophages, foreign body giant cells and lymphocytes in joints and around them; 4) interstitial urate crystal deposits in renal parenchyma and 5) Urolithiasis.

Hyperuricemia, which is the basic biochemical characteristic and a prior condition for gout is defined as a presence of urate plasma concentration higher than 420 $\mu$mol/L (7.0 mg/dL) being a proof of increase of total urate body concentration.

In this stage (which occurs only in 2 to 18% of gouty patients), uric acid is high but without arthritis. In men it starts on puberty while in women is usually present until menopause; in patients with enzymatic defects it may start from birth. It may last along life and generally a gouty arthritis condition is present after 20 years of hyperuricemia, although 10 to 40% from hyperuricemic patients will show urolithiasis until 10 years before the first acute articular crisis.

Hyperuricemia may appear as a consequence of a high urate synthesis, reduction in uric acid removal or a combination of both mechanisms.

When hyperuricemia is present, plasma and extracellular liquids are urate oversaturated, favoring crystal formation and deposition on tissues. These factors lead to symptoms included within gout concept.

Uric acid is the end product of purine degradation in human being. It is a weak acid, with a pKa from 5.75 to 10.3. Urates are an ionized form of uric acid and predominate in plasma, extracellular liquid and synovial liquid, in such a way that about 98% thereof is in monosodium urate form, in presence of a pH of 7.4.

Monosodium urate is easily ultrafiltered and dialyzable from plasma. Urate attachment to plasma proteins has limited physiological meaning. Plasma is saturated with monosodium urate in a concentration of 415 $\mu$mol/L (6.8 mg/dL) at 37° C. Therefore, upon higher concentrations plasma is urate oversaturated and there is a possibility of urate crystal precipitation.

However, precipitation is not produced even before urate plasma concentrations as high as 4800 $\mu$mol/L (80 mg/dL). Why urate forms stable oversaturated solutions in plasma and at the same time may have connection with the presence of solubilizing substance presence is unknown.

Uric acid is more soluble in urine than in water, probably because of the presence of urea, proteins and mucopolysaccharides. Urine pH remarkably has influence in solution. In the presence of a pH of 5.0, urine is saturated with uric acid in concentrations between 360 and 900 $\mu$mol/L (6 a 15 mg/dL). With a pH of 7.0, saturation is achieved with concentrations between 9480 and 12000 $\mu$mol/L (158 to 200 mg/dL). Uric acid ionized forms in urine comprise mono- and disodium urates, potassium urate, ammonium urate and calcium urate.

Although the synthesis of purine nucleotides and their rupture takes place in every tissue, urate is only synthesized in tissues comprising xanthine oxidase, especially liver and small intestine.

The amount of body urates is the net result obtained between the produced amount and the excreted amount. Urate synthesis varies as a function of purine content in diet and biosynthesis speed, degradation and purine savings. Under normal conditions, between two thirds and three fourth parts of synthesized urate is removed in kidneys and a large portion of remaining urate in intestine.

A triple mechanism for renal control of uric acid in human being is proposed comprising: glomerular filtration, tubular secretion and tubular reuptake.

From 8 to 12% of filtered urate in glomerulus is excreted in urine in the form of uric acid. Urate is reabsorbed after filtering from 98 to 100%. About half of reabsorbed urate is again secreted towards the proximal tubule, wherein 40 to 44% is again reabsorbed.

Urate plasma concentration varies as a function of age and sex. Most of children show a concentration between 180 and 240 $\mu$mol/L (3.0 to 4.0 mg/dL).

Concentration increases during man puberty, but it remains low in women until menopause. Although not fully understood the cause of this variation depending on sex, this may be partly due to a larger urate functional excretion in women, as a consequence of hormonal influence.

Mean urate plasma values in adult men and premenopausal women are 415 and 360 $\mu$mol/L (6.8 and 6.0 mg/dL), respectively. After menopause, women values are raised up to about those from men.

In adults, concentrations are stably increased along time and they vary depending on height, body weight, blood pressure, renal function and alcohol consumption.

Hyperuricemia may be defined as an urate plasma concentration higher than 420 $\mu$mol/L (7.0 mg/dL). This definition is based on physicochemical, epidemiologic and disease-related criteria.

From a physicochemical point of view, hyperuricemia is urate blood concentration exceeding solubility limits in plasma monosodium urate, that is, 415 $\mu$mol/L (6.8 mg/dL).

Hyperuricemia is defined in epidemiologic studies by a mean plus two standard deviations of the values determined in a health random population. In a broad study, 95% from unselected subjects show urate plasma concentrations below 420 $\mu$mol/L (7.0 mg/dL).

Finally, hyperuricemia may be defined when related with the risk of developing a disease. Gout or urolithiasis risk increases with urate concentrations higher than 420 $\mu$mol/L (7.0 mg/dL), and grows proportionally as said figure is increased. Hyperuricemia shows a prevalence between 2.0 and 13.2% in ambulatory regime adults and somehow higher in hospitalized patients.

_Hyperuricemia causes_. Hyperuricemia is usefully classified as to subjacent physiology; that is, depending on: a synthesis increase, a clearance reduction or a combination thereof.

_Enhanced urate synthesis_. Diet represents a purine exogenous source and therefore, contributes to urate plasma concentration proportionally to purine content. A strict restriction in purine intake reduces mean urate plasma concentration in only 60 $\mu$mol/L (1.0 mg/dL) and uric acid urinary removal in about 1.2 mmol/day (200 mg/day).

Since about 50% from ingested RNA purines and 25 % of ingested DNA purines appear in urine in uric acid form, foods with high nucleic acid content possess a significant effect over urate urinary concentration. Within said foods are liver, sweetbreads (thymus and panchreas), kidneys and anchovies.

Endogenous sources for _purine synthesis_ also have influence in urate plasma concentration.

Purine de novo biosynthesis comprising forming a purine ring from linear structures is an eleven-step process which ends in inosate formation (IMP).

The first step combines phosphoribosylpyrophosphate (PRPP) and glutamine and is catalyzed by amidophosphoribosyltransferase enzyme (amidoPRT). Biosynthesis purine speed and subsequent urea synthesis mainly depends on said enzyme. AmidoPRT is regulated, by a substrate, PRPP, which favors reaction progress and by biosynthesis end products, IMP and other ribonucleic products exerting a negative feedback.

Another regulating path is purine base rescue by hypoxanthine phosphoribosyltransferase enzyme (HPRT). Said enzyme catalyzes a combination of hypoxanthine and guanine purine bases with PRPP forming the respective IMP and GMP ribonucleotides.

An increase in rescue activity thus delays de novo synthesis, reducing PRPP concentrations and increasing those for inhibiting ribonucleotides.

Urate plasma concentration is closely related with purine de novo biosynthesis speed. A disorder linked to X which causes an increase in PRPP synthetase enzyme activity increases PRPP synthesis and accelerates de novo biosynthesis. Subjects who show this congenital metabolic disorder have a purine overproduction, hyperuricemia and hyperaciduria, thus showing uric acid stones and gouts before 20 year old.

_Uric acid decreased excretion._ Up to 98% of subjects with primary hyperuricemia and gout show uric acid renal control disorder. This is demonstrated by the presence of a decreased urate removal speed, regarding to glomerular filtration speed (or regarding to insulin removal speed) over a wide series of filtered loads. Consequently, subjects with gout remove about 40% less uric acid than normal subjects for any urate plasma concentration.

Uric acid excretion is increased in subjects with gout and in those not suffering it when urate plasma concentration increases as a consequence of purine intake or intravenous administration, but in the first group urate plasma concentration must be from 60 to 120 $\mu$mol/L (1 to 2 mg/dL) higher than normal to achieve an equivalent uric acid removal speed.

Uric acid excretion alteration may theoretically appear as a consequence of a reduced tubular secretion or an increased tubular reuptake. Urate filtration reduction does not seem to cause primary hyperuricemia, but contributes to renal failure hyperuricemia.

_Hyperuricemia complications._ Although gouty disease symptoms may follow almost any combination as to their manifestation, typical sequence consists of progression from asymptomatic hyperuricemia, acute gouty arthritis, intercritical gout, chronic or tophaceous gout. Nephrolytiasis may appear before or after the first gouty arthritis event.

It is estimated that hyperuricemia prevalence is between 2.0 and 13.2%, while gout prevalence varies between 1.3 and 3.7% in all population. The more urate plasma concentration, the higher probability that an individual suffers from gout.

In a broad study, gout incidence was 4.9% for subjects with urate concentrations of 540 $\mu$mol/L (9.0 mg/dL) or more, compared with 0.5% for those with concentrations between 415 and 535 $\mu$mol/L (7.0 and 8.9 mg/dL). Similarly, gout complications are correlated with hyperuricemia duration and severity. Most part of gouty arthritis initial events are present after 20 to 40 years of sustained hyperuricemia, with a maximum commencement age between 40 and 60 years in men and after menopause in women.

In gouty arthritis, gout characteristic feature is monoarticular arthritis acute attacks. First attack commences explosively and is one of the most painful events ever experienced. Subjects occasionally report prodromes or prior episodes of mild pain lasting hours. Acute gout severe pain is accompanied by intense inflammation signs: swelling, erithema, heat and hypersensitivity.

Inflammation may be accompanied by febricula. If treatment is not received, attack may reach a maximum from 24 to 48 hours after the first symptoms and eases after 7 to 10 days. Skin over affected zone may be flaked as episode is solved.

Typically, initial attack only affects to one joint although there are polyarticular events, which may be more frequent in women.

Gouty arthritis affects primarily to peripheral joints, specifically lower limbs. Further, certain locations may be affected such as fascia plantar, Achiles tendon insertion or other tenosynovial.

The first metatarsophalangeal joint is affected in more than 50% of initial attacks and in 90% of subjects at any time. A sacro-iliac condition from sternal or spinal handle is seldom seen.

Any factor which causes an increase or a sudden decrease in urate plasma concentration may cause an acute attack; the higher correlation being established with factors causing a fast decrease.

Theoretically, sudden increases in urate concentration may cause crystal formation, while a decrease in urate plasma and extracellular concentration may lead to partial dissolution and previously formed crystal destruction.

Other cause factors are: stress, traumatisms, infections, hospitalization, surgery, fasting, weight reduction, overfeeding, excessive meals, alcohol and drugs. Hospitalization and drugs are possibly the most significant cited factors.

Gout acute attacks are present in 20 to 86% from subjects with gout background when they are hospitalized due to medical or surgical causes.

Stress representing a severe disease, medication change, liquid and electrolyte movements and general anesthesia, probably contribute to said exacerbation.

Attacks may be present after using thiacidic diuretics causing hyperuricemia or after introducing treatments with Allopurinol or other drugs for reducing plasma urate.

Acute gouty arthritis attacks are occasionally present in the absence of hyperuricemia. Most of said attacks may be probably explained by the presence of factors reducing urate plasma concentration temporarily modifying commonly present hyperuricemia (which may have caused the attack).

Theoretically, gout may be developed at any time when synovial liquid is oversaturated with urate. Free water is removed faster from articular space than urate, so that if synovial liquid amount is increased comprising a normal urate concentration as a consequence of a traumatism or edema, urate intraarticular concentration temporarily increases when initial problem is solved and water is removed faster.

Urate oversaturated concentrations may cause the formation of crystals and precipitate an attack.

Acute gout appears as a consequence of, and interaction between urate crystals and polymorphonuclear leukocytes, comprising humoral and cellular inflammatory mechanism activation. Urate crystals activate the complement through both classical and alternative path. Hageman factor and the coagulation contact system are also activated, which leads to bradykinin, calicrein and plasmin synthesis. Urate crystal interaction with neutrophils lead to a release of lysosomal enzymes, free radicals derived from oxygen, leukotriene metabolites and prostaglandins, collagenase and protease.

Crystal phagocytosis by neutrophils causes a crystal-induced chemotactic factor release (CCF).

CCF, leukotrine B$_4$ (LTB$_4$) and component activated by C5a complement are all chemotactic factors and contribute to a response of polymorphonuclear leukocytes during the acute arthritis initial stage.

Mononuclear phagoytic substitute to polymorphonuclear cells along time. Urate crystals cause a prostaglandin (PGE$_2$), lysosomal enzyme alpha tumor necrosis factor and interleukin-1 and 6 (IL-1 and IL-6) release by said cells.

Synovial coating cells also participate in inflammatory response through a release of inflammatory mediators.

Inflammatory potential of urate crystals is affected largely by the presence of absorbed proteins. Purified absorbed IgG causes a platelet secretion induced by crystals, an increase of superoxide synthesis and an increase of lysosomal enzymes from polymorphonyclear leukocytes.

Tophii are monohydrated monosodium urate crystal aggregates, generally surrounded by giant cells because of a mononuclear cellular inflammatory reaction of foreign body. They may be formed in articular or extraarticular structures causing soft and hard tissue deformity and destructuration. They may lead to cartilage and bone destruction in joints which unchains secondary degenerative changes.

Once that gouty arthritis diagnosis is ensured, therapeutic agents shall be managed such as: non-steroidal anti-inflammatories and xanthine oxidase inhibitors.

Currently, most of drugs found in marketplace for treatment of gouty disease or gout, comprise active principles which are independently formulated, which meet a specific therapeutic activity; however, these drugs are generally administered at very high doses, causing severe secondary effects.

## SUMMARY OF INVENTION

Development of present invention was carried out in order to provide different pharmaceutical alternatives intended to reduce symptomatology in patients suffering from gouty disease or gout such as: pain and inflammation, and which also reduce urate levels and decrease the risk of showing secondary effects by an administration of high drug doses, whereby several pharmaceutical compositions comprising a combination of a non-steroidal anti-inflammatory agent and a xanthine oxidase enzyme inhibiting agent are disclosed, those which act synergistically and are formulated in a single dosage unit to be orally administered, providing benefits such as: lower active principle concentrations comprised in the formulation, smaller administered doses, therapeutic effect enhancement, faster pharmacological action, risk reduction of manifesting severe complications and a risk decrease that secondary effects are present.

## DETAILED DESCRIPTION OF INVENTION

**Non-steroidal anti-inflammatory agents** (NSAIDs) include a large group of drugs showing a significant analgesic, anti-inflammatory and antipyretic activity, in addition to other therapeutic effects.

Enolic acids are found within the NSAIDs classification being also subdivided in: pyrazolones (Metamizol), Pyrazolydindiones (Phenylbutazone), Oxicams (Piroxicam and Meloxicam). Within said classification another drug group called *Coxibs* may be also found, which includes: Celecoxib, Parecoxib, Lumiracoxib, Etoricoxib, Rofecoxib (withdrawn) and Valdecoxib (withdrawn).

Non-steroidal antiinflammatories show their analgesic action by relieving pain associated with inflammation or with a tissue lesion by decreasing prostanoid production which sensibilize nociceptors to mediators, such as bradykinin. They are efficient in mild to moderate intensity pains. Their antipyretic effect is shown by prostaglandin production inhibition in hypothalamus and interference with temperature regulating mechanisms.

Their anti-inflammatory activity is produced by reducing the components of the inflammatory response wherein COX products perform a significant role such as: vasodilatation, edema and pain.

All non-steroidal antiinflammatories are analgesic and antipyretic, some (indomethacin, piroxicam) are more anti-inflammatory, most are moderately anti-inflammatory (ibuprofen, nabumetone) and others (paracetamol) have a minimum anti-inflammatory effect.

Some non-steroidal antiinflammatories have platelet antiaggregating activity, which is of special interest in the case of the acetylsalicylic acid because of its platelet COX irreversible inhibiting effect. This NSAID is of great use in prevention of coronary and brain thromboembolic accidents.

Similarly, some NSAID have uricosuric action as a consequence of uric acid transport inhibition from renal tubule lumen to interstitial space. It is only appreciated with some NSAIDs to high doses, such as phenylbutazone, sulfinpyrazone or salicylates.

Under NSAID action mechanisms, all their effects are related witch cyclooxygenase inhibition (COX) and with prostaglandin production inhibition. AAS is the only which produces a COX-1 irreversible inhibition. NSAID anti-inflammatory effect is clearly related with COX-2 inhibition and many of the undesirable effects are related with COX-1 inhibition.

**Meloxicam** was characterized as a powerful anti-inflammatory agent in several inflammation conventional models. Moreover, it demonstrated a weak gastric ulcerogenicity in rat stomach in spite of its powerful anti-inflammatory activity.

When these results were initially obtained, there was no explanation of the best pharmacological profile of Meloxicam compared with conventional NSAID. At that time, only one COX (cyclooxygenase) was known, the enzyme responsible of prostaglandin synthesis, and it was thought that inhibition of the activity of this enzyme was responsible of both therapeutic and side effects of NSAIDs.

COX inhibition, and thus the prevention of prostaglandin formation, provided a unified explanation of NSAID action regarding their therapeutic actions as well as their gastrotoxicity, nephrotoxicity and antithrombotic effects.

Upon discovering a second COX enzyme, COX-2, an hypothesis about NSAID anti-inflammatory effect achieved through a mechanism different than that with frequently observed compound side effects has been proposed, including an alteration of stomach cytoprotection, renal function and platelet aggregation inhibition. COX-1 is the constitutive isoenzyme found under physiological conditions in most tissues, a called "maintenance" enzyme while COX-2 expression is mostly induced, particularly during inflammatory processes. However, recent evidence indicates that COX-2 expression is also constitutive in some tissues such as CNS and kidney.

Most of available NSAID inhibit both enzymes non-selectively which causes typical gastrointestinal anti-inflammatory effects (related with COX-1 inhibition).

From that on, Meloxicam has been demonstrated to inhibit more powerfully COX-2 than COX-1 enzyme at recommended anti-inflammatory doses, which explains the initial experimental results: Meloxicam showed in the range of relevant pharmacological models a dose-dependant COX-2 selective inhibition compared to COX-1; while COX-1 inhibition was incomplete, platelet aggregation inhibition was not demonstrated at Meloxicam recommended doses.

Meloxicam is an enolcarboxamidic derivative belonging to enolic acid group. It performs a powerful inhibiting activity over cyclooxygenase-2 (COX-2), with a selectivity 75 times higher for COX-2 than for COX-1, participating as a prostaglandin synthesis inhibitor having the function of being mediators responsible of inflammatory processes.

Meloxicam was described in 1994 as a COX-2 selective inhibitor as to COX-1. 5-methyl group present in Meloxicam thiazolyl ring may enter into COX-2 active site additional space, which explains part of its selectivity. Effective intracellular access is determined by its specific lipophylic and amphiphylic properties. Meloxicam has a membrane solubility in its acidic form 10-fold higher than Piroxicam, in comparison with other NSAIDs.

Meloxicam leaves from membranes approximately twice faster than Diclofenac. Generally, meloxicam is rapidly transported through membranes, but within a range which allows an efficient interaction with its target enzyme. Low meloxicam hydrosolubility at acid pH and its amphyphylic protonation behavior are responsible of tissular kinetics which prevents a high concentration of this drug in certain digestive tube tissues.

Therefore, Meloxicam does not show a typical "ionic entrapment" behavior characterizing to NSAIDs of carbonic acid class, which may contribute to a gastrointestinal tolerance favorable profile clinically observed.

Meloxicam has a good digestive absorption and an optimum bioavailability (89%), after being only administered in a single oral dose. Some of the main pharmacokinetic characteristics are: its prolonged absorption, sustained serum concentrations and long clearance half-life (20 hours), which allows administration of a single daily dose. Once absorbed in digestive tract, Meloxicam is easily diffused towards blood and inflammated tissues, having a high adherence with plasma proteins (>99%) and its metabolites are excreted both in urine and in feces.

Celecoxib is a selective cyclooxygenase-2 enzyme (COX-2) inhibitor with anti-inflammatory properties similar to other non-steroidal anti-inflammatory drugs (NSAIDs) such as naproxen or diclofenac. Due to its specificity towards cyclooxygenase-2, the risk of producing side effects at gastrointestinal level is lower than with conventional NSAIDs.

Celecoxib is a cyclooxygenase-2 enzyme (COX-2) non-competitive inhibitor unlike conventional NSAIDs which are COX-1 and COX-2 inhibitors. These enzymes catalyze arachidonic acid conversion to prostaglandin H2 and to tromboxanes. COX-2 is important in substance synthesis which participate as inflammation and pain mediators, while COX-1 produces prostaglandins which are useful for gastric and renal functions. Non-selective NSAIDs such as Ibuprofen or Diclofenac inhibit two cyclooxygenase types, being Celecoxib about 100 times more active with COX-2 than with COX-1.

At concentrations which are reached in plasma and target organs, Celecoxib does not significantly inhibit COX-1. Any effect is neither present over platelet aggregation. COX-2 expression is induced by a number of cytokines and growth factors in inflammated tissues. In vitro studies have shown that COX-2 inhibitors, whether selective or not, have preventive activity over colon cancer.

Celecoxib is a drug to be orally administered. After oral dose administration, Celecoxib is well absorbed reaching maximum plasma levels in about 3 hours. Foods with high fat content delay Celecoxib absorption from 1 to 2 hours and increase drug amount absorbed in 10 to 20%; therefore, this drug may be administered with food.

Celecoxib is bound extensively to plasma proteins (especially albumina) and is broadly distributed, being distribution volume about 400 L. Celecoxib is metabolized through P450 cytochrome CYP 2C9 enzymatic system, having identified three inactive metabolites in human plasma. Only a small part of Celecoxib without altering is recovered in urine and feces.

Metabolites are removed through renal or biliary route: 57% of dose is recovered in feces and 27% in urine. Plasmatic clearance is about 500 mL/min, and clearance half life is about 11 hours. At old age, liver failure or renal dysfunction significantly affect Celecoxib pharmacokinetics, with ethnic and race factors having also influence.

**Xanthine oxidase enzyme inhibiting agents** decrease uric acid production blocking the final step in urate synthesis, while oxypurines (xanthine and hypoxanthine) are increased.

Uric acid is the main product of endogenous or exogenous purine catabolism (diet) in men. It is a weak acid found in monosodium urate form at body liquid pH and temperature. Most part of uric acid production takes place in liver and intestinal mucose which dispose xanthine oxidase (XO) enzyme. Then it passes into blood where a concentration from 2.5 to 6.8 mg/dL is reached, depending on age (increased up to 20 years) and on sex (V>M), assumedly by estrogen uricosuric action. 50% of total uric acid from body is daily removed and replaced, 2/3 of uric acid is removed by kidney and the remaining 1/3 by bile and other digestive secretions when passed to intestine and suffering from uricase enzyme (urico-oxidase) action of intestinal bacteria. From 98 to 100 % of filtered urate by glomerulus, is reabsorbed. 50% thereof is secreted in distal lengths and most of it is later reabsorbed (post-secretory reabsorption) in such a way that appears in urine only in 10% of initially filtered uric acid.

When extracellular urate concentration exceeds a critical level, urate body fluid saturation is produced being capable of precipitating in a number of tissues in body (mainly at joint level) in the form of monosodium urate crystals.

The presence of intraarticular urate crystals may be asymptomatic or may unchain an inflammatory process by complement activation, kinin system and cytokine release (interleukin-1, interleukin-6, interleukin-8, TNF-alpha) by macrophages and synoviocytes. Endothelium activation and IgG and ClQ absorption by crystals, are key mechanisms in this process.

The repeated monosodium urate crystal deposition leads to tophii appearance (urate crystal aggregates), which cause a foreign body mononuclear cellular granulomatous inflammatory reaction, leading to secondary degenerative changes and chronic arthritis.

Allopurinol is a hypoxanthine-analog pyrazolopyrimidine, functioning as a potent competitive inhibitor of xanthine oxidase enzyme. It is the more used antihyperuricemic agent being also a substrate for said enzyme. Oxypurinol, the main Allopurinol metabolite, is also an efficient inhibitor of xanthine oxidase enzyme.

Allopurinol is absorbed in digestive system and has a bioavailability between 80 to 90%. Allopurinol half-life is from 1 to 2 hours and that for oxypurinol is 21 hours. Oxypurinol excretion increases with urixosuric agents and reduces renal failure. Allopurinol results efficient in treatment of all types of hyperuricemia, but is specifically indicated in the following cases: 1) gout patients, 2) urate overproduction signs (uric acid in 24 hour urine higher than 4.8 mmol (800 mg) with normal diet, or higher than 3.6 mmol (600 mg) with a purine restriction diet) and 3) patients with renal stones comprising 2, 8-dihydroxyadenine.

Allopurinol administration reduces urate plasma concentration and uric acid urinary excretion along the first 24 hours, with a maximum reduction at two weeks.

About 20% of ingested Allopurinol is excreted in stools. While Allopurinol is cleared by glomerular filtration, oxypurinol is reabsorbed by renal tubules in a similar form as uric acid. Oxypurinol clearance is increased by uricosuric drugs and consequently, the association of an uricosuric drug with Allopurinol reduces its effects over xanthine oxidase and increases uric acid excretion in urine.

Its action mechanism is because it leads to oxypurinol, a xanthine oxidase inhibitor which also inhibits purine de novo synthesis, since Allopurinol is a xanthine oxidase enzyme substrate to which it inhibits competitively. Allopurinol acts over purine catabolism without a biosynthesis modification. It reduces uric acid production by inhibiting biochemical reactions which lead to its formation. Allopurinol is a structural analog of hypoxanthine natural purinic base, acting as a xanthine oxidase inhibitor, the enzyme which is responsible of converting from hypoxanthine to xanthine and from xanthine to uric acid, the end product of purine catabolism in humans.

Uric acid concentration reduction favors precipitate dissolution (tophii), prevents the appearance of acute attacks and avoids the appearance of severe complications. The possibility of forming uric acid stones practically disappears and hence, nephropathy appearance.

Allopurinol efficient dose is 300 mg/day. However, the required dose for controlling suitably urate plasma concentration depends on the severity of tophii disease and renal function.

Allopurinol may be administered once a day thanks to prolonged oxypurinol half-life.

Nowadays there are in marketplace a number of pharmaceutical products targeted for treatment of gouty disease or gout, wherein a number of active principles are found which are independently formulated; however, in this kind of pathologies prevention of severe complications is of key importance, as well as rapidly reducing symptomatology in patients suffering from said disease; therefore, the activity of a drug group acting through different routes is indispensable for achieving recovery in patients undergoing said disorder.

Pharmaceutical compositions which are subject of present invention comprise a synergistic combination of a non-steroidal anti-inflammatory agent, such as active principle: Meloxicam or Celecoxib and a xanthine oxidase enzyme inhibiting agent, such as active principle: Allopurinol, in addition to pharmaceutically acceptable excipients, those which are formulated in a single dosage unit to be orally administered, which are indicated for control and treatment of Gouty disease or gout, Gouty arthritis and other related pathologies.

Said pharmaceutical compositions have been developed taking into account that above mentioned active principles have a great efficacy and capability for control and treatment of gouty disease or gout, gouty arthritis and other related pathologies, and since these drugs act synergistically, they achieve a reduction in symptomatology characterizing to said pathologies, such as inflammation and pain, reducing plasma urate concentrations, in addition to increase the speed of its pharmacological action by administering lower active principle concentrations, maximizing its therapeutic effect with smaller administered doses, reducing the risk of presenting severe complications and decreasing the risk of manifesting side effects.

One of the non-steroidal anti-inflammatory agents used in pharmaceutical compositions subject of the present invention, such as active principle: Meloxicam, is present in the formulation in a concentration range from 7.5 mg to 45.0 mg per dose unit.

Another non-steroidal anti-inflammatory agent used in pharmaceutical compositions subject of present invention, such as active principle: Celecoxib, is present in the formulation in a concentration range from 100.0 mg to 600.0 mg per dose unit.

Xanthine oxidase enzyme inhibiting agent used in the pharmaceutical composition subject of the present invention, such as active principle: Allopurinol, is present in the formulation in a concentration range from 100.0 mg to 800.0 mg per dose unit.

In order to assess the efficiency of the pharmaceutical composition subject of the present invention, as well as the synergistic effect resulting from a combination of active principles: Allopurinol and Meloxicam in a single dosage unit, a comparative clinical study was carried out wherein above mentioned active principles were separately administered, as well as a combination thereof.

## CLINICAL STUDY FOR ASSESSING THE EFFICACY AND SYNERGISTIC

## EFFECT OF A COMBINATION OF ALLOPURINOL/MELOXICAM.

### PHASE I.

### Materials and Methods.

A clinical study was carried out assessing the efficacy of a combination of Allopurinol/Meloxicam vs independent delivery of Allopurinol and Meloxicam in patients with Gouty arthritis.

There were 150 patients with gout and with uric acid levels at least of 8 mg/dL randomly selected for this study.

Main validation criterion was uric acid concentration of 6 mg/dL, in the last 3 monthly determinations. Secondary validation criteria included a reduction of incidence in gout worsening events.

Patients were divided into three treatment groups with a follow-up of 52 weeks.

Group 1 received: Allopurinol 300 mg per day.
Group 2 received: Meloxicam 15 mg per day.
Group 3 received: the combination of Allopurinol 300 mg/Meloxicam 15 mg

### Results

There were not significant differences as to baseline characteristics in patients (Table 1).

Table 1 Baseline characteristics

|  | Group 1 (n=50) | Group 2 (n=50) | Group 3 (n=50) |
|---|---|---|---|
| Age | 45 ± 7 | 47 ± 4 | 42 ± 8 |
| Sex F/M | 5/45 | 8/42 | 10/40 |
| Uric Acid > 7 | 100% | 100% | 100% |
| Pain | 100% | 100% | 100% |

Main validation criterion was reached in most of patients who received a combination of Allopurinol / Meloxicam, reducing 10 mg/dL baseline to 6 mg/dL at the end of study in average; patients who received only Allopurinol improved from a baseline average of 9 mg/dL to 7 mg/dL at the end of the study and patients who received only Meloxicam, from 10 mg/dL baseline to 9 mg/dL at the end (Table 2).

Table 2. Baseline and final data for uric acid concentrations

|  | Group 1 (n=50) | Group 2 (n=50) | Group 3 (n=50) |
|---|---|---|---|
| Uric Acid Average | 9/7 mg/dL | 10/9 mg/dL | 10/6 mg/dL |

Although the acute event incidence decreased with continuous treatment, global incidence along 9 to 52 weeks was better in the group which received the combination and similar in groups 1 and 2.

Six adverse events were reported in treatment Group 1 with gastrointestinal upset such as fullness sensation; 12 patients from Group 2 reported epigastric gastrointestinal pain and fullness sensation, and 5 adverse events were reported in Group 3 with gastrointestinal upset such as nausea and gastrointestinal pain. In none of the groups was necessary to cancel the treatment, nuisances disappeared along weeks, and drug was recommended to be administered during main meals.

## Conclusions

The study demonstrated that the combination of Allopurinol / Meloxicam was effective for treatment of gout acute attacks, significantly reducing both symptomatology and blood urate levels, although in many cases was sufficient the use a NSAID together with diet, in these patients the use of drugs which achieve an urate reduction is fundamental, especially when uric acid levels are high.

It is worth to mention that treatment of these cases shall be for long term, to prevent relapses and to achieve a good uric acid level maintenance.

**PHASE II.**

In order to define the possibility of an antinociceptive synergistic interaction, Meloxicam antinociceptive effects were determined acting as a non-steroidal anti-inflammatory (NSAID) and from Allopurinol, acting as a xanthine oxidase enzyme inhibitor, administered both separately and in combination.

An arthritic pain model in rat was determined for said purpose. Data were interpreted by a Surface Synergistic Interaction analysis (ISS) and an isobolographic analysis to determine the interaction nature. ISS was calculated from the total antinociceptive effect produced by the combination after subtracting the antinociceptive effect produced by drug separately. Female rats only received oral Meloxicam, only oral Allopurinol, or 24 different combinations of Meloxicam plus Allopurinol.

## Material and methods.

For this study Wistar female rats were used [Crl (WI) BR] weighing between 180 to 200 gr. Feed was maintained during 12 hours before experimentation with water ad libitum. All experimental procedures followed the recommendations from the Committee for Research and Ethical Issues of the International Association for the Study of Pain (Covino et al., 1980) and Guidelines on Ethical Standards for Investigations of Experimental Pain in Animals (Zimmermann, 1983), and they were carried out according to a protocol approved by the local Animal Ethics Committee. Number of animals in experiment was kept minimal and animals were kept in a room with climate and light control with a 12-hour light/dark cycle.

*Drugs*

Uric acid was suspended in mineral oil; Meloxicam and Allopurinol were dissolved in carboxymethylcellulose and orally administered.

## Study design.

Antinociceptive effects produced by Meloxicam and Allopurinol whether administered individually or in combination were studied. Firstly, each Meloxicam (0.18, 0.32, 0.56, 1.0, 1.78, 3.16 or 5.62 mg/kg) or Allopurinol (3.16, 5.62, 10.0, 17.78, 31.62, 56.23 or 100.0 mg/kg) dose was delivered to six animals to obtain the corresponding dose-response curves. Meloxicam (0.10, 0.18, 0.32, 0.56, 1.0 or 1.78 mg/kg) and Allopurinol (3.16, 5.62, 10.0 or 17.78 mg/kg) dose were then combined for analyzing the possible synergistic interactions (24 combinations in total). At the completion of study rats were sacrificed.

*Measurement of antinociceptive activity*

Antinociceptive activity was determined by using the PIFIR model which has been described in detail (López-Munoz et al., 1993b). Animals were anesthesized with ether in a chamber (Pyrex glass dryer with ether vapor). Nociception was induced through a 0.05 mL uric acid intraarticular (ia) injection suspended in mineral oil in knee joint from right back leg. The suspension was prepared by milling 3.0 g. of uric acid with 10 mL of mineral oil in a glass mortar with pestle (Pyrex). Intraarticular injection was carried out through patellar ligament using a 1 mL glass syringe (Beckton Dickinson LTA, Brazil) with a 5 mm 24-gauge needle. An electrode was implanted immediately after to the plantar surface of each back claw between plantar pads. Rats were allowed to recover from anesthesia and then arranged in a stainless steel cylinder which was rotated at 4 rpm, forcing rats to walk for 2 minute periods every 30 minutes along 6.5 hours. A training period was not necessary since rats learned it in the first minute. Contact time between each electrode from rat limb and cylinder was registered. After uric acid injection, rats progressively developed a damaged limb dysfunction. Contact time of damaged limb reached a zero value after 2.5 hours from uric acid injection application; in this time Meloxicam and Allopurinol were independently or jointly administered. This time was determined as zero time for measuring antinociceptive effects; these effects were measured every 30 minutes during the following 4 hours. This allowed determining the time course for antinociceptive effects in the same animal. Antinociception was considered as a recovery of contact time. Data are expressed as Functionality percentage index (IF%, that is, contact time on injected leg by the contact time in left leg, control, multiplied by 100). For the purpose of this study, nociception induction in experimental animals was unavoidable. However, care was taken to prevent an unnecessary suffering. All experiments were performed between 7:00 am and 2:00 pm.

*Gastrointestinal* effect measurement.

Wistar female rats (between 150 and 180 g body weight) were subject to fasting 24 hours before experiment. Indomethacin was supplied (20 mg/kg) to cause gastric ulcer at 100% (Lee et al., 1971; Déciga-Campos et al., 2003).

Meloxicam (1.0 mg/kg), Allopurinol (17.8 mg/kg), vehicle (0.5% carboxymethylcellulose) and the combination of Meloxicam plus Allopurinol (1.0 and 17.8 mg/kg, respectively) were orally administered thereafter at the same time and to the five groups (six rats each). About 2.5 hours later, all groups received a second administration of the same doses. Stomachs were examined 5 hours after the first treatment in the following way: animals were sacrificed and stomach was separated which was opened along a minor curve, mildly rinsed with formaldehyde (2%) and examined. Severity of gastric lesions induced with drug was calculated as a ratio from injury number (stomach ulcer or erosion) caused by treatment provided and the number of lesions caused by indomethacin (100%). This was considered to reflect side effects induced by drug. The sum of area from all body ulcers in each animal body was calculated and served as ulcerating index. Gastric injury percentage was calculated in the following way:

$$\% \text{ gastric injury } = (IUM/IUI) \times 100$$

wherein IUM is Drug Ulcerating index on testing ($mm^2$) and IUI is assayed indomethacin ulcerating index ($mm^2$).

## Data presentation and statistical assessment

Study data, tables and figures are expressed as IF%. Curves for IF% vs time were prepared for each treatment and the corresponding course time was obtained. Antinociception was estimated as a recovery of IF%. Accumulated antinociceptive effect during the whole observation period (4 hours) was determined as the area below the curve (ABC) from time course to obtain a dose-response curve and for analyzing the total antinociceptive effect obtained by analgesic agent whether alone or in combination.

Synergism between Meloxicam and Allopurinol was calculated through an analysis of Synergistic Interaction surface (SIS) and with the isobolographic method (Tallarida et al., 1989). ABC was calculated for each drug combination and for each of the components. Over the addition base of the individual pharmacological effects (Seegers et al., 1981) an ABC equivalent to the sum was expected. If the sum of the individual corresponding ABC was higher than the theoretical sum, the result was considered enhancement; when it was similar to the theoretical sum then was determined to show additive antinociceptive effects. ABC was obtained through a trapezoidal rule (Rowland and Trozer, 1989). All values for each treatment are average $\pm$ S. E. M., for six animals. ABC values for drug combinations were compared with expected values using the Student test. Obtained ABC values from antinociceptive effects produced by Meloxicam or Allopurinol (assayed apart) were compared with ABC values obtained from the corresponding combination through the variance analysis' (ANOVA) and Dunnett test.

Gastrointestinal side effects caused by, Meloxicam or Allopurinol (whether assayed separately or in combination) were obtained with gastrointestinal effects obtained from Indomethacin through Dunnett test. A $P < 0.05$ was determined as statistically significant.

Doses which caused 50% of maximum possible effect ($DE_{50}$) of each drug were calculated by performing a linear regression analysis of the linear portion from dose-response curves. The isobologram was prepared by using the $DE_{50}$ when drugs were delivered alone or in combination. In order to perform the isobolographic analysis, Meloxicam and Allopurinol were delivered in combination as fixed rates of equivalent $DE_{50}$ dose in efficacy for each drug (Meloxicam= 1:1). $DE_{50}$. values ($\pm$ S. E. M.) for Meloxicam and Allopurinol were drafted over "x-" and "y~" axes respectively, and theoretical additive point was calculated according to Tallarida et al., (1989). $DE_{50}$ value from combination total dose was calculated from the dose-response curve of combined drugs. Statistical significance between additive theoretical point and $DE_{50}$ experimental value was validated by using the Student test. A $DE_{50}$ experimental figure significantly lower than $DE_{50}$ theoretical addition (P<0.05) was considered to indicate a synergistic interaction between Meloxicam and Allopurinol.

**Results**

*Uric acid and vehicle effects.*

Uric acid induced a complete dysfunction in right back leg corresponding to a zero IF% value along 2.5 hours. This dysfunction was kept along the whole experimental period lasting 4 hours. Rats receiving vehicle (0.5% carboxymethylcellulose) did not show any significant IF% recovery along the observation period. Meloxicam used doses (0.18-5.62 mg/kg) and Allopurinol (3.16-56.23 mg/kg) did not affect the walking capability of treated rats with said drugs (data are not shown. Allopurinol at 100 mg/kg, did not cause side effects in animals, neither effects in walking capability of rats.

*Antinociceptive effects of individually assayed drugs.*

Dose-response curves for Meloxicam and Allopurinol. Both drugs increased ABC in a dependant-dose form but they showed a different efficacy (Meloxicam produced the maximum efficacy). Therefore, Meloxicam (2.16 mg/kg) showed a great antinociceptive efficacy of 295.8 $\pm$ 14.9 au and Allopurinol (56.23 mg/kg) showed an antinociceptive efficacy of 207.7 $\pm$ 24.7 au. $DE_{50}$ values for drugs indicate that there were significant differences in their antinociceptive potencies: Meloxicam ($DE_{50}$= 0.86 $\pm$ 0.10 mg/kg) was more potent than Allopurinol ($DE_{50}$= 44.29 $\pm$ 0.06 mg/kg). There were not adverse effects with used doses.

*Antinociceptive effects of drug combinations*

Antinociceptive effect of the 24 combinations over three-dimensional graphs. These were constructed using an average of six animals for each dose whether alone or combined. Maximum antinociceptive effect which may be obtained in several Meloxicam + Allopurinol combinations (1.78 + 17.78 mg/kg, respectively) was 372.7 $\pm$ 15.6 au. Statistical data analysis indicate an interaction between Meloxicam and Allopurinol (P<0.05) while no antagonist effects were found in the assayed combination.

Results in order to discriminate between additive and enhancement effects. They were calculated from the total antinociceptive effect produced by combinations after produced antinociceptive effect subtraction for each component alone. Results higher than "0" level were determined to show enhancement, while those at "0" level were determined as addition. Although this mapping type allows an observation of antagonist antinociceptive effects, these were not obtained in present study. At the same time, 14 combinations of Meloxicam + Allopurinol produced antinociceptive effects and 10 produced enhancement with reliability limits of 95% (P<0.05), these combinations were: 17.78 mg/kg Allopurinol with either, 0.18, 0.32 or 0.56 mg/kg Meloxicam; 10.0 mg/kg Allopurinol with either 0.18, 0.32, 0.56 or 1.78 mg/kg Meloxicam; 5.62 mg/kg Allopurinol with either 0.32 or 0.56 mg/kg Meloxicam and 3.16 mg/kg Allopurinol with 0.32 mg/kg de Meloxicam. In order to obtain the surface of synergistic interaction of Meloxicam + Allopurinol combinations, all interaction points were unified in a plane. The result is a synergistic interaction surface with these shown analgesic drugs. It is easy to visualize Meloxicam + Allopurinol drug interactions (i.e., addition or enhancement). For instance, 10 combinations of Meloxicam + Allopurinol showed different enhancement degrees of antinociceptive effects, but 2 combinations of Meloxicam + Allopurinol showed high enhancement effects (0.32 $\pm$ 10 and 0.56 + 10 mg/kg, respectively). Allopurinol at 10.0 mg/kg dose provided an ABC of 22.5 $\pm$ 9.3 au and Meloxicam, at a 0.32 mg/kg dose provided an ABC of 65.0 $\pm$ 9.6 au; however, the combination of Meloxicam + Allopurinol (10 + 0.32 mg/kg) allowed an AUC of 233.5 $\pm$ 25.8 au, which is higher than expected ABC resulting in the sum of individual values (i.e., 87.5 au) (P<0.001). Emax analysis of the corresponding time course curves showed an increase in obtained values from combinations (86.6 $\pm$ 6.3%) which was higher than corresponding values (Meloxicam 33.7 $\pm$ 8.7% and Allopurinol 17.1 $\pm$ 9.1%) obtained from the arithmetic sum (50.8%).

Antinociceptive effects obtained by combinations that produced the maximum antinociceptive effect are: 1.78 mg/kg Meloxicam + 17.78 mg/kg Allopurinol, and the combination that produced the highest enhancement is: 0.56 mg/kg Meloxicam + 10.0 mg/kg Allopurinol. Antinociception produced by Meloxicam + Allopurinol (1.78 + 17.78 mg/kg) represented the maximum antinociceptive effect (which represents a full recovery) obtained with 372.7 $\pm$ 15.6 au, while Meloxicam independently administered (1.78 mg/kg) showed an ABC of 279.0 $\pm$ 15.5 au, and Allopurinol independently administered (17.78 mg/kg) produced only 58.2 $\pm$ 23.7 au. This result was significant when considering that maximum Meloxicam used dosage (3.16 mg/kg) produced lower antinociceptive effect: 295.8 $\pm$ 14.9 au. Disclosed combination (0.56 mg/kg Meloxicam + 10.0 mg/kg Allopurinol) only represents a combination which produced the highest enhancement in antinociceptive effect (169.4% more ABC or full antinociceptive effect being the sum of individual ABC); similarly, both the course time and obtained ABC with this combination was higher (P<0.001) than with he obtained respective values with the sum of individual agents (121.2 au). Antinocicpetion produced by a combination of Meloxicam / Allopurinol (0.56 + 10.0 mg/kg) was 290.6 $\pm$ 27.7 au, while Meloxicam delivered alone (0.56 mg/kg) showed an ABC of 98.7 $\pm$ 15.5 au and Allopurinol administered alone (10.0 mg/kg) showed an ABC of only 22.5 $\pm$ 9.3 au. A significant antinociceptive effect was obtained with the combination during the whole observation period (4 hours). Another approach for synergistic interaction research between the two selected analgesic drugs is the isobolographic method, Tallarida et al., 1989. Isobologram showed an antinociceptive interaction of Meloxicam and Allopurinol in the rat pain-induced functional impairment model. Experimental point is far below the additive line, indicating an additive synergism (P<0.05).

*Measurement of gastrointestinal side effects.*

Allopurinol administration did not cause ulcers or erosions. Its side effects were similar to those from vehicle. However, Meloxicam generated a lower ulcer area (13.7 $\pm$ 2.7 mm$^2$) and lower erosion number (15.0 $\pm$ 4.9) than Indomethacin (P<0.05), which was considered as the most harmful component in terms of injury number and severity caused in stomach (i,e., ulcers and erosions) (100%). The combination of Meloxicam + Allopurinol generated less ulcers (16.9 $\pm$ 6.9 mm$^2$) and a smaller number of erosions (5.0 $\pm$ 1.4) than Indomethacin (P<0.05). It is interesting that the combination of Meloxicam + Allopurinol reduced erosion generation (P< 0.05) and ulcerations were similar to those from Meloxicam administered alone.

## Conclusions

The combination Meloxicam + Allopurinol showed and efficient synergistic effect, compared with the independent administration of said drugs, on the other hand, showed lower gastrointestinal and ulceration side effects.

The combination may be administered in diseases such as gout, gouty arthritis and other related pathologies.

## NOVELTY OF INVENTION

Having disclosed the present invention, the following content is considered novel and therefore claimed as property.

## Claims

1. Pharmaceutical compositions **characterized in that** comprise a synergistic combination of a non-steroidal anti-inflammatory agent, such as active principle: Meloxicam or Celecoxib, and a xanthine oxidase enzyme inhibiting agent, such as active principle: Allopurinol, in addition to pharmaceutically acceptable excipients; wherein said active principles are present in the formulations in a concentration range comprising from 7.5 mg to 45.0 mg for Meloxicam, from 100.0 mg to 600.0 mg for Celecoxib and from 100.0 mg to 800.0 mg for Allopurinol; which are formulated in single dosage units to be orally administered, those indicated for control and treatment of gout, gouty arthritis and other related diseases.

2. Pharmaceutical composition according to claim 1, **characterized in that** comprises a synergistic combination of a non-steroidal anti-inflammatory agent, such as active principle: Meloxicam, and a xanthine oxidase enzyme inhibiting agent, such as active principle: Allopurinol, in addition to pharmaceutically acceptable excipients, those which are formulated in a single dosage unit.

3. Pharmaceutical composition according to claim 2, **characterized in that** the non-steroidal anti-inflammatory agent, such as active principle: Meloxicam, is present in the formulation in a concentration range comprising from 7.5 mg to 45.0 mg, being preferably used in the formula a concentration from 7.5 mg to 15.0 mg per dose unit.

4. Pharmaceutical composition according to claims 2 and 3, **characterized in that** xanthine oxidase enzyme inhibiting agent, such as active principle: Allopurinol, is present in the formulation in a concentration range comprising from 100.0 mg to 800.0 mg, being preferably used in the formula a concentration from 100.0 mg to 300.0 mg per dose unit.

5. Pharmaceutical composition according to claims 2 to 4, **characterized in that** is formulated in a single dosage unit to be orally administered in capsule or tablet form.

6. Pharmaceutical composition according to claims 2 to 5, **characterized in that** is indicated for control and/or treatment of gouty disease or gout, gouty arthritis and other related pathologies.

7. Pharmaceutical composition according to claim 1, **characterized in that** comprises a synergistic combination of a non-steroidal anti-inflammatory agent, such as active principle: Celecoxib, and a xanthine oxidase enzyme inhibiting agent, such as active principle: Allopurinol, in addition to pharmaceutically acceptable excipients, those which are formulated in a single dosage unit.

8. Pharmaceutical composition according to claim 7, **characterized in that** the non-steroidal anti-inflammatory agent, such as active principle: Celecoxib, is present in the formulation in a concentration range comprising from 100.0 mg to 600.0 mg, being preferably used in the formula a concentration of 100.0 mg per dose unit.

9. Pharmaceutical composition according to claims 7 and 8, **characterized in that** xanthine oxidase enzyme inhibiting agent, such as active principle: Allopurinol, is present in the formulation in a concentration range comprising from 100.0 mg to 800.0 mg, being preferably used in the formula a concentration of 300.0 mg per dose unit.

10. Pharmaceutical composition according to claims 7 to 9, **characterized in that** is formulated in a single dosage unit to be orally administered in capsule or tablet form.

11. Pharmaceutical composition according to claims 7 to 10, **characterized in that** is used for control and/or treatment of gouty disease or gout, gouty arthritis and other related pathologies.

12. Pharmaceutical compositions according to claims 1 to 11, **characterized in that** they manifest an important efficacy for control and treatment of inflammation and pain, in addition to ensure a reduction in plasma urate levels present in patients who suffer gouty disease or gout, gouty arthritis and other related diseases, also achieving a higher rapidity on its pharmacological action in shorter time with smaller administered doses, as well as a maximization of a therapeutic effect, reduction in risks of showing severe complications and a decrease in risk of showing secondary effects.

# INTERNATIONAL SEARCH REPORT

| | |
|---|---|
| International application No. | |
| PCT/ MX 2008/000123 | |

### A. CLASSIFICATION OF SUBJECT MATTER

see extra sheet

According to International Patent Classification (IPC) or to both national classification and IPC

### B. FIELDS SEARCHED

Minimum documentation searched (classification system followed by classification symbols)

A61K

Documentation searched other than minimum documentation to the extent that such documents are included in the fields searched

Electronic data base consulted during the international search (name of data base and, where practicable, search terms used)

INVENES,EPODOC, WPI, EMBASE, BIOSIS, NPL, MEDLINE, XPESP, HCAPLUS

### C. DOCUMENTS CONSIDERED TO BE RELEVANT

| Category* | Citation of document, with indication, where appropriate, of the relevant passages | Relevant to claim No. |
|---|---|---|
| X | WEAVER, A.L.:"Acute gout-risk factors and inappropiate therapy". The American Journal of Medicine, 2006, vol. 119 (11A), pages S9-S12, page S9, primera column, segundo paragraph. | 1-12. |
| X | PLEURY, B.:"Drugs used to treat muscle and joint disease". Anaesthesia and Intensive Care Medicine, 2006, vol. 7, número 3, pages 104-106, page 104, page 105, column 1, lines 8-14. | 1-12. |
| X | LIOTE, F. ET AL.:"Traitement of the goutte". Revue du Rhumatisme, 2007, vol. 74, pages 160-167, page 161, paragraph 3.2, page 162, paragraph 4.2.1. | 1-12. |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 19 December 2008        (19.12.2008) | (07/01/2009) |
| Name and mailing address of the ISA/ O.E.P.M. Paseo de la Castellana, 75 28071 Madrid, España. Facsimile No.   34 91 3495304 | Authorized officer H. Aylagas Cancio Telephone No. +34 91 349 8563 |

Form PCT/ISA/210 (second sheet) (July 2008)

## INTERNATIONAL SEARCH REPORT

International application No.

PCT/MX 2008/000123

C (continuation). DOCUMENTS CONSIDERED TO BE RELEVANT

| Category* | Citation of documents, with indication, where appropriate, of the relevant passages | Relevant to claim No. |
|---|---|---|
| X | CROFT, J.D.:"Discussion following cases 4 and 5". The American Journal of Medicine, 2006, vol. 119, (11A), pages S16-S19, page S16, último paragraph, page S17, tab the 1. | 1-12. |
| X | VAN DOORNUM, S. ET AL.:"Clinical manifestations of gout and their management". Medical Journal of Australia, 2000, vol. 172, 10, pages 493-497, pages 496, 497. | 1-12. |
| A | CHENG, T. ET AL.:"A single-blind, randomized, controlled trial to assess the efficacy and tolerability of rofecoxib, diclofenac sodium, and meloxicam in patients with acute gouty arthritis". Clinical Therapeutics, 2004, vol. 26, (3), pages 399-406, page 399. | 1-12. |

Form PCT/ISA/210 (continuation of second sheet) (July 2008)

## INTERNATIONAL SEARCH REPORT

International application No.

PCT/ MX 2008/000123

CLASSIFICATION OF SUBJECT MATTER

*A61K 31/415* (2006.01)
*A61K 31/5415* (2006.01)
*A61K 31/519* (2006.01)
*A61P 19/06* (2006.01)
*A61P 29/00* (2006.01)

Form PCT/ISA/210 (extra sheeet) (July 2008)